# EUROPÄISCHE PATENTANMELDUNG

(11) **EP 0 847 995 A2**
(43) Veröffentlichungstag der Anmeldung: **17.06.1998**
(21) Anmeldenummer: 97120816.0
(22) Anmeldetag: 27.11.1997
(51) Int. Cl.: C07D 239/30

(54) **Verfahren zur Herstellung von 2,4,6-Trichlorpyrimidin**

(30) Priorität: 10.12.1996 DE 19651310
(71) Anmelder: BAYER AG, 51368 Leverkusen (DE)
(72) Erfinder: Käss, Volker, Dr., 51375 Leverkusen (DE)

(57) **Zusammenfassung**

Verfahren zur Herstellung von 2,4,6-Trichlorpyrimidin, dadurch gekennzeichnet, daß man in einem ersten Reaktionsschritt Barbitursäure gegebenenfalls in Gegenwart eines Katalysators mit Phosphoroxychlorid und anschließend in einem zweiten Reaktionsschritt mit Phosphorpentachlorid oder mit dieses bildende Reaktanden, insbesondere Phosphortrichlorid und Chlor, bei einer Temperatur von 20 bis kleiner 80°C umsetzt.

## Beschreibung

Die vorliegende Erfindung betrifft ein neues Verfahren zur Herstellung von 2,4,6-Trichlorpyrimidin.

2,4,6-Trichlorpyrimidin kann nach einem Verfahren gemäß Chem. Ber. **92** (1959) 2937 durch Umsetzung von Barbitursäure mit Phosphoroxychlorid (POCl₃) in Gegenwart von Dimethylanilin hergestellt werden. Das so erhaltene 2,4,6-Trichlorpyrimidin wird wäßrig aufgearbeitet. Die Ausbeute, bezogen auf Barbitursäure, beträgt 85 % der Theorie. Dieses Verfahren ist jedoch mit Nachteilen verbunden. So ist beispielsweise für einen großtechnischen Prozeß die wäßrige Aufarbeitung des 2,4,6-Trichlorpyrimidin enthaltenden Reaktionsgemisches unerwünscht, da dies sicherheitstechnisch problematisch ist und in der Regel eine mehrfache Extraktion der wäßrigen Phase erforderlich wird. Durch die wäßrige Aufarbeitung entstehen zudem große Abwassermengen mit hoben Phosphat- und Amingehalten. Darüber hinaus ist in dem Verfahren eine große Menge des tertiären Amins nötig (etwa 1,8 Mol pro Mol Barbitursäure), die wiedergewonnen werden muß.

Wird eine geringere Menge Dimethylanilin eingesetzt, als bei dem Verfahren von J. Baddiley et al. J. Chem. Soc. 1944, 678 (etwa 0,8 Mol pro Mol Barbitursäure), sinkt die Ausbeute auf 59 %. Ist für weitere Umsetzungen das 2,4,6-Trichlorpyrimidin als wasseifreies Edukt erforderlich, ist eine anschließende aufwendige Entwässerung nicht zu umgehen, bei der das Produkt teilweise hydrolysiert.

Es wurde ein Verfahren zur Herstellung von 2,4,6-Trichlorpyrimidin gefunden, das dadurch gekennzeichnet ist, daß man in einem ersten Reaktionsschritt Barbitursäure, gegebenenfalls in Gegenwart eines Katalysators, mit Phosphoroxychlorid (POCl₃) und anschließend in einem zweiten Reaktionsschritt mit Phosphorpentachlorid (PCl₅) oder mit dieses bildende Reaktanden, insbesondere Phosphortrichlorid (PCl₃) und Chlor, bei einer Temperatur von 20 bis kleiner 80°C umsetzt.

Es ist vorteilhaft, wenn man anschließend das gebildete und das nicht umgesetzte Phosphoroxychlorid und das 2,4,6-Trichlorpyrimidin von dem Reaktionsgemisch abtrennt, insbesondere destillativ.

Das insbesondere durch Destillation abgetrennte Phosphoroxychlorid kann beispielsweise dazu verwendet werden, erneut in dem erfindungsgemäßen Verfahren eingesetzt zu werden.

Überraschenderweise wurde gefunden, daß der erste Reaktionsschritt bereits bei niedrigen Temperaturen und ohne Gasentwicklung abläuft. Bereits bei Raumtemperatur, besonders aber bei einer Temperatur von 50 bis kleiner 80°C, vorzugsweise von 60 bis 75°C, erfolgt die Umsetzung mit POCl₃. Bei höheren Temperaturen läuft der 1. Reaktionsschritt zwar geringfügig schneller ab, führt jedoch in der Regel schon nach kurzer Zeit zu zähen unlöslichen Ausfällungen von Polyphosphorverbindungen. Dieses Festwerden des Reaktionsgemisches verhindert insbesondere eine technische Durchführung.

In einer bevorzugten Ausführungsform des erfindungsgemäßen Verfahrens läuft der 1. Reaktionsschritt ohne HCl-Entwicklung ab. Besonders bevorzugt ist das zusätzliche Einleiten von HCl in einer Menge von 1 bis 6 Mol pro Mol Barbitursäure. Die dafür eingesetzte HCl kann beispielsweise dem 2. Reaktionsschritt, bei dem sie gebildet wird, entnommen werden.

Der 2. Reaktionsschritt wird ebenfalls bei einer Temperatur von 20 bis kleiner 80°C, vorzugsweise bei 60 bis Kleiner 80°C, insbesondere bei 60 bis 75°C durchgeführt.

Das Phosphoroxychlorid wird vorzugsweise in einer Menge von 3 bis 15 Mol, insbesondere von 4 bis 7 Mol, bezogen auf 1 Mol Barbitursäure, eingesetzt.

Größere Mengen als 15 Mol, bezogen auf 1 Mol Barbitursäure, an Phosphoroxychlorid können zwar eingesetzt werden, bieten aber keinen besonderen Vorteil. Kleinere Mengen als die für die vollständige Umsetzung erforderlichen 3 Mol je Mol Barbitursäure führen zu geringeren Ausbeuten und gegebenenfalls zu unrührbaren Reaktionsgemischen.

Phosphoroxychlorid übernimmt vorzugsweise die Funktion eines Lösungsmittels, so daß das erfindungsgemäße Verfahren vorzugsweise ohne zusätzliche Lösungsmittel durchgeführt werden kann.

Die jeweiligen Reaktionsschritte können jedoch auch in Gegenwart eines inerten Lösungsmittels ablaufen. Ebenfalls vorteilhaft ist es, das Verfahren in Abwesenheit von Wasser durchzuführen. Der gegebenenfalls im ersten Reaktionsschritt, als auch im zweiten Reaktionsschritt gebildete Chlorwasserstoff entweicht und kann beispielsweise mit Hilfe eines Wäschers aus der Abluft entfernt werden.

Für den im ersten Reaktionsschritt gegebenenfalls anwesenden Katalysator sind beispielsweise organische Basen geeignet, wie tertiäre Amine, beispielsweise Triethylamin, Tripropylamin, Tri-n-Butylamin, Dimethylanilin, Diethylanilin, N,N-Di-ethylmethylanilin, N-Ethyl-diisopropylamin, Trioctylamin, Triisobutylamin, 1,8-Bis-(Dimethylamino)-naphthalin, N,N-Dimethyl-p-toluidin oder ähnliche, außerdem N-substituierte Carbonsäure- und Sulfonsäureamide oder N,N-disubstituierte Carbonsäure- und Sulfonsäureamide, wie N,N-Dimethylformamid, N,N-Diethylformamid, N,N-Dimethylacetamid, N,N-Diethylacetamid, N-Formylpiperidin, Tetramethylharnstoff, 1-Alkyl-2-pyrrolidone, wie beispielsweise 1-Methyl-2-pyrrolidon (NMP), 1-Octyl-2-pyrrolidon oder 1-Dodecyl-2-pyrrolidon, Dibutylformamid und Methyl-Stearyl-Formamid sowie basische Heterocyclen, wie Pyridin, 2-Methylpyridin, 3-Methylpyridin, 4-Methylpyridin, 2-Ethylpyridin, 4-Ethylpyridin, 2,4-Dimethylpyridin, 2,6-Dimethylpyridin, 3,5-Dimethylpyridin, 5-Ethyl-2-methylpyridin, 2,4,6-Trimethylpyridin, 4-(Dimethylamino)-pyridin, 4-(1-Pyrolidinyl)-pyridin und Chinolin; natürlich können auch Mischungen der genannten Katalysatoren eingesetzt werden.

Gegebenenfalls verwendete Katalysatoren werden vorzugsweise in einer Menge von weniger als 0,1 Mol, insbesondere weniger als 0,03 Mol, pro Mol Barbitursäure eingesetzt, wobei größere Mengen als 0,1 Mol pro Mol Barbitursäure keinen besonderen Vorteil bieten.

Im zweiten Reaktionsschritt erfolgt die Umsetzung vorzugsweise mit 2,0 bis 5 Mol Phosphorpentachlorid oder dieses bildende Reaktanden, insbesondere mit 3,0 bis 3,3 Mol Phosphortrichlorid und 2,4 bis 3,5, insbesondere 2,9 bis 3,2 Mol Chlor pro Mol Barbitursäure. Die Umsetzung des erfindungsgemäßen Verfahrens verläuft insbesondere dann günstig, wem Phosphortrichlorid und Chlor in stöchiometrischen Mengen eingesetzt werden; d.h. pro Mol Barbitursäure werden 3 Mol Chlor und 3 Mol Phosphortrichlorid eingesetzt. Vorteilhaft ist es, das Phosphortrichlorid ganz oder teilweise, vor oder simultan mit dem Chlor zuzudosieren. Vorzugsweise werden Phosphortrichlorid und Chlor simultan der Reaktionsmischung zudosiert. Chlorgas wird dabei vorzugsweise durch ein Gaseinleitungsrohr direkt in die Reaktionsmischung eingeleitet. Besonders vorteilhaft ist es, wenn während der Reaktion kein Chlorüberschuß auftritt. Ein Überschuß an Chlor ist zwar möglich, bietet aber keine besonderen Vorteile, sondern führt im Gegenteil zu mehr Aufwand bei der Abgasentsorgung. Ein geringfügiger Überschuß an PCl₃ ist vorteilhaft. Ein deutlicher PCl₃-Überschuß ist für die eigentliche Reaktion des 2. Reaktionsschrittes nicht störend, jedoch sind daraus resultierende höhere PCl₃-Gehalte im POCl₃ bei der Wiederverwendung von POCl₃ für einen weiteren Reaktionsansatz gegebenenfalls nachteilig. Das mittels Destillation wiedergewonnene POCl₃ besitzt vorzugsweise einen PCl₃-Gehalt von ≤5 %, insbesondere <1 %. Ein PCl₅-Überschuß ist zwar möglich, führt aber in der Regel durch Sublimation während der Produktdestillation zu unerwünschten Ablagerungen in der Destillationskolonne und im Abgassystem.

Die Ausbeute an 2,4,6-Trichlorpyrimidin, das nach dem erfindungsgemäßen Verfahren erhalten wird, liegt im allgemeinen bei 90 bis 94 % der Theorie, bezogen auf Barbitursäure. Das so erhaltene 2,4,6-Trichlorpyrimidin ist im wesentlichen frei von 2,4,5,6-Tetrachlorpyrimidin sowie von Wasser. Eine Chlorierung in der 5-Position des Pyrimidins wurde unter den erfindungsgemäßen Verfahrensbedingungen nicht beobachtet. Das erfindungsgemäße Verfahren bietet gegenüber den bis jetzt bekannten Verfahren mehrere Vorteile. Neben einer höheren Ausbeute ist das 2,4,6-Trichlorpyrimidin in technisch außerordentlich einfacher Weise aus dem Reaktionsgemisch destillativ in sehr reiner Form zu gewinnen. Zudem fällt bei einer vorzugsweise nichtwäßrigen Aufarbeitung Abwasser erst gar nicht an.

Das nach dem erfindungsgemäßen Verfahren entstandene Produkt kann beispielsweise destillativ aus dem Reaktionsgemisch gewonnen werden. So isoliertes 2,4,6-Trichlorpyrimidin kann gegebenenfalls bis zu 1 % an Phosphorverbindungen enthalten. Für den Fall, daß besonders hohe Reinheitsgehalte gefordert werden, beispielsweise für die Herstellung von Pflanzenschutzprodukte, ist es vorteilhaft, die erhaltene Reaktionsmischung zunächst von POCl₃ destillativ zu befreien und dann bei einer Temperatur von etwa 170°C zu tempern, vorzugsweise etwa 2 Stunden.

Das anschließend durch Destillation gewonnene Produkt besitzt eine hohe Reinheit.

Das Tempern kann bei verschiedenen Temperaturen unterschiedlich lange durchgeführt werden, beispielsweise 2 bis 4 Stunden bei 150 bis 170°C oder 4 bis 6 Stunden bei 130 bis 150°C.

2,4,6-Trichlorpyrimidin ist bekannt als wichtiges Zwischenprodukt für die Herstellung von Reaktivfarbstoffen und Pflanzenschutzwirkstoffen und als Ausgangsprodukt für die Herstellung von weiteren wichtigen Pyrimidinderivaten wie z.B. 2,4,6-Trifluorpyrimidin.

### Beispiele

### Beispiel 1

In vorgelegte 3500 kg (32,2 Mol) Phosphoroxychlorid wurden 520 kg (4,06 Mol) Barbitursäure und 10 kg N-Methylpyrrolidon zugegeben, abschließend wurde auf 75 +/- 5°C aufgeheizt. Bei dieser Temperatur war keine Abgasentwicklung zu beobachten, der Ansatz blieb zudem eine Suspension. Nach einer Nachrührzeit von 7 Stunden wurden 1675 kg (12,2 kMol) Phosphortrichlorid zugegeben und bei 75 +/- 5°C 850 kg (12,1 kMol) Chlor eingeleitet. Dabei fand eine kräftige Gasentwicklung statt. Nach beendeter Chlorierung wurde Phosphoroxychlorid (4815 kg) unter Normaldruck abdestilliert. Nach Zugabe eines Ditolylether-Gemischs als Destillationshilfsmittel wurde dann eine POCl₃-2,4,6-Trichlorpyrimidin-Fraktion (270 kg) abdestilliert, die in die Folgepartie wiedereingesetzt werden kann. Danach wurde 2,4,6-Trichlorpyrimidin im Vakuum destilliert. Ausbeute 685 kg 2,4,6-Trichlorpyrimidin entsprechend ca. 93 % der Theorie.

### Beispiel 2 Simultandosierung

Zu vorgelegten 3500 g (23,2 Mol) Phosphoroxychlorid wurden 512 g (4 Mol) Barbitursäure und 10 g N-Methylpyrrolidon zugegeben, abschließend wurde auf 75 +/- 5°C aufgeheizt. Nach einer Nachrührzeit von 7 Stunden wurden simultan 1650 g (12,0 Mol) Phosphortrichlorid und 840 g (11,8 Mol) Chlor zugegeben bzw. eingeleitet. Nach destillativer Aufarbeitung wurden 661 g 2,4,6-Trichlorpyrimidin entsprechend 90 % der Theorie Ausbeute erhalten.

### Beispiel 3 Temperung vor Produkt-Destillation

Zu vorgelegten 3500 g Phosphoroxychlorid wurden 512 g (4 Mol) Barbitursäure und 10 g N-Methylpyrrolidon zugegeben, abschließend wurde auf 75 +/- 5°C aufgeheizt. Nach 7 Stunden wurden 1650 g Phosphortrichlorid zugegeben und bei 75 +/- 5°C Chlor eingeleitet. Nach beendeter Chlorierung wurde Phosphoroxychlorid unter Normaldruck abdestilliert und nach Zugabe eines Destillationshilfsmittels dann im Vakuum eine POCl₃-2,4,6-Trichlorpyrimidin-Fraktion abdestilliert. Nach Belüften wurde der Destillationsrückstand 2 Stunden bei 170°C gerührt. Anschließend wurde im Vakuum erst das bei der Temperung neugebildete POCl₃ und danach 2,4,6-Trichlorpyrimidin abdestilliert. Ausbeute 673 g 2,4,6-Trichlorpyrimidin entsprechend ca. 91,7 % der Theorie abdestilliert. Das auf diese Weise gewonnene Produkt ist besonders rein (Gehalt 99,8 %) und enthält Phosphorverbindungen nur in Spuren.

### Beispiel 4 Zusatz von HCl-Gas

Zu vorgelegten 3500 g (23,2 Mol) Phosphoroxychlorid wurden 512 g (4 Mol) Barbitursäure und 10 g Triethylamin zugegeben, abschließend wurde auf 75 +/- 5°C aufgeheizt. Bei dieser Temperatur wurden 182 g Chlorwasserstoff eingeleitet. Anschließend wurden 1650 g Phosphortrichlorid zugegeben und 840 g Chlor eingeleitet. Nach destillativer Aufarbeitung wurden 668 g 2,4,6-Trichlorpyrimidin entsprechend ca. 91,0 % der Theorie erhalten.

## Patentansprüche

1. Verfahren zur Herstellung von 2,4,6-Trichlorpyrimidin, dadurch gekennzeichnet, daß man in einem ersten Reaktionsschritt Barbitursäure, gegebenenfalls in Gegenwart eines Katalysators, mit Phosphoroxychlorid und anschließend in einem zweiten Reaktionsschritt mit Phosphorpentachlorid oder mit dieses bildende Reaktanden, insbesondere Phosphortrichlorid und Chlor, bei einer Temperatur von 20 bis kleiner 80°C umsetzt.

2. Verfahren gemäß Anspruch 1, dadurch gekennzeichnet, daß man den ersten Reaktionsschritt bei einer Temperatur von 50 bis kleiner 80°C, vorzugsweise von 60 bis 75°C und den zweiten Reaktionsschritt bei einer Temperatur von 60 bis Kleiner 80°C, vorzugsweise bei 60 bis 75°C durchführt.

3. Verfahren gemäß Anspruch 1, dadurch gekennzeichnet, daß man im zweiten Reaktionsschritt das Phosphortrichlorid ganz oder teilweise vor oder simultan mit dem Chlor zudosiert.

4. Verfahren gemäß Anspruch 3, dadurch gekennzeichnet, daß man für die Umsetzung des zweiten Reaktionsschrittes Phosphortrichlorid und Chlor simultan zur Reaktionsmischung zudosiert.

5. Verfahren gemäß Anspruch 1, dadurch gekennzeichnet, daß man als Katalysator tertiäre Amine, N-substituierte Carbonsäure- und Sulfonsäureamide oder N,N-disubstituierte Carbonsäure- und Sulfonsäureamide, oder basische Heterocyclen verwendet.

6. Verfahren gemäß Anspruch 1, dadurch gekennzeichnet, daß man als Katalysator Triethylamin, Tributylamin, N,N-Dimethylformamid oder 1-Methyl-2-Pyrrolidon verwendet.

7. Verfahren gemäß Anspruch 1, dadurch gekennzeichnet, daß man pro Mol Barbitursäure 2,5 bis 5 Mol, insbesondere 3,0 bis 3,3 Mol, Phosphortrichlorid und 2,4 bis 3,5 Mol, insbesondere 2,9 bis 3,2 Mol, Chlor einsetzt.

8. Nach dem Verfahren gemäß Anspruch 1 erhaltene 2,4,6-Trichlorpyrimidine.

9. Verwendung der gemäß Anspruch 1 erhaltenen 2,4,6-Trichlorpyrimidine zur Herstellung von 2,4,6-Trifluorpyrimidin.

10. Verfahren gemäß Anspruch 1, dadurch gekennzeichnet, daß in dem ersten Reaktionsschritt HCl eingeleitet wird.
